# EUROPEAN PATENT APPLICATION

(11) **EP 4 029 455 A1**
(43) Date of publication of application: **20.07.2022**
(21) Application number: 20880197.7
(22) Date of filing: 23.10.2020
(51) Int. Cl.: A61B 10/00, A61B 3/113, A61B 5/11, A61B 5/12

(54) **HEAD-POSITION SWAY MEASURING DEVICE, HEAD-POSITION SWAY MEASURING METHOD, AND BIOLOGICAL INFORMATION ACQUISITION SYSTEM USING SAID DEVICE AND METHOD**

(30) Priority: 24.10.2019 JP 2019193335
(71) Applicant: Keio University, Tokyo, 108-8345 (JP)
(72) Inventor: FUJIOKA Masato, Tokyo 160-8582 (JP); KASEDA Kaori, Tokyo 160-8582 (JP); YAMANOBE Yoshiharu, Tokyo 160-8582 (JP); OGAWA Kaoru, Tokyo 160-8582 (JP)
(74) Representative: Schön, Christoph
(86) International application number: PCT/JP2020/039838
(87) International publication number: WO 2021/079970

(57) **Abstract**

The head-position sway measuring device (D) includes a touch panel (1a) that gives the instruction to open or close eyes, an acceleration sensor (2a) that measures a displacement of a head position, and a sway recognition unit (1b) that recognizes a head-position sway value based on the displacement of the head position. The acceleration sensor (2a) measures a first measurement value, which is a measurement value obtained in the eye-open state, and then measures a second measurement value, which is a measurement value obtained in the eye-closed state. The sway recognition unit (1b) recognizes the head-position sway value based on the first measurement value and the second measurement value.

## Description

### Technical Field

The present invention relates to a head-position sway measuring device and a head-position sway measuring method for measuring a head-position sway value, which indicates the variation amount of sway of the head position of a subject, and a biological information acquisition system using the same.

### Background Art

A dizzy spell is when you feel as if you or something around you is moving or spinning when in fact it is not (hereinafter referred to simply as "dizziness"). There are many causes of dizziness, but it is often caused by abnormalities in the inner ear organs such as the semicircular canals.

Therefore, in general, methods that measure the state of the inner ear organs and sway of body (especially head position) are effective in diagnosing diseases that produce dizziness. For that, there are known methods of assisting diagnosis by measuring the sway and referring to the measurement values during diagnosis (see, for example, Patent Literature 1).

### Citation List

### Patent Literature

Patent Literature 1: Japanese Patent Laid-Open No. 2017-121512

### Summary of Invention

### Technical Problem

As for the diseases that produce dizziness, in some autosomal recessive genetic diseases, the state of the abnormality of the inner ear organs (inner ear disorders) varies irregularly. However, there are many unknowns about its frequency, the range of variations in hearing acuity threshold based on these abnormality, and the duration of exacerbation.

However, with the measurement devices employed in conventional diagnostic aids such as those described in Patent Literature 1, it is difficult to accurately grasp the condition of the inner ear disorders that varies irregularly.

An object of the present invention, which was made in consideration of the aforementioned points, is to provide a head-position sway measuring device and a head-position sway measuring method that can accurately acquire information on the condition of dizziness, and a biological information acquisition system using the device and method.

### Solution to Problem

The present invention is a head-position sway measuring device that measures a head-position sway value that is a value indicating a variation amount of sway of a head position of a subject, the device comprising:
an opening/closing instruction unit that instructs the subject to open or close his or her eyes;
a displacement measurement unit that measures a displacement of the head position of the subject; and
a sway recognition unit that recognizes the head-position sway value based on the measured displacement of the head position, wherein
the displacement measurement unit measures one of a first measurement value that is a measurement value of the displacement of the head position in a state where the opening/closing instruction unit instructs to open eyes and a second measurement value that is a measurement value of the displacement of the head position in a state where the opening/closing instruction unit instructs to close eyes, and measures the other of the first measurement value and the second measurement value successively or after a lapse of a predetermined length of time, and
the sway recognition unit recognizes the head-position sway value based on the first measurement value and the second measurement value related to the first measurement value.

Thus, the head-position sway measuring device of the present invention instructs the subject to open or close his or her eyes through the opening/closing instruction unit, and measures the first measurement value and the second measurement value, which are the measurement values of the displacement of the head position obtained in the state where the instruction to open or close the eyes is given, and recognizes the head-position sway value based on these measurement values.

At this time, the first measurement value and the second measurement value are measured successively or after a predetermined length of time (i.e., a length of time predetermined by the designer) has elapsed. For this reason, the first measurement value and the second measurement value are necessarily measured with the same posture except for the eye-open or closed state.

The head-position sway value obtained by this head-position sway measuring device is based on the first measurement value and the second measurement value measured in this way, and are therefore more accurate than those obtained by conventional measurement devices. Therefore, reference to the head-position sway value obtained with this head-position sway measuring device allows information indicating the condition of dizziness to be obtained more accurately.

In the present invention, the opening/closing instruction unit preferably includes an image display unit that displays an instruction image representing an instruction to the subject to open or close his or her eyes.

In general, during measurement of head-position sway, subjects tend to look at objects (such as a clock in a room) around them or not to look at anything, unless they are looking at some object. As a result, their gaze wanders, causing a risk of body deviation in the subject.

For this reason, if the subject is instructed to open or close his or her eyes through the instruction image, the subject can be made naturally gaze at the instruction image, the gaze of the subject can be fixed (and his or her posture is therefore stabilized), and the body deviation of the subject can be controlled. Thus, the head-position sway value and therefore information indicating the condition of dizziness can be obtained more accurately.

Also, in the present invention, in the case where the instruction image is displayed,
the instruction image preferably includes a predetermined graphic image and a background image that serves as a background of the graphic image, and
a boundary part of the graphic image adjacent to the background image preferably has a gradation of at least one of brightness and saturation changing gradually from a periphery toward a center of the graphic image.

If the boundary line of the graphic image that the subject gazes at during the head-position sway measurement is clear, under the influence of the boundary line, the sway control function due to visual stimulus may be exerted excessively and the sway due to dizziness may be controlled.

If the boundary part has the gradation, such excessive exertion of the sway control function can be controlled. Thus, the head-position sway value and therefore information indicating the condition of dizziness can be obtained more accurately.

Also, in the present invention, in the case where the instruction image includes a graphic image and a background image,
the graphic image is preferably a circular or polygonal image, and
the value of the at least one of brightness and saturation of the graphic image preferably increases from the periphery toward the center and, in the central area, decreases toward the center.

Here, "circular" shape includes not only circles but also ellipses and other generally circular shapes. Also, "polygonal" shape includes not only those with straight sides, but also those with the sides curved toward the periphery.

With this configuration, the graphic image is an image of a graphic with a blurred periphery and a blurred central area (i.e., an image that looks like a ring). As a result, the gaze of the subject is naturally guided to the center of the graphic image, and can therefore be firmly fixed, and unnecessary body deviation can be efficiently controlled.

Also, a biological information acquisition system of the present invention comprises:
at least one of a hearing acuity measuring device that measures a hearing acuity threshold of the subject, a nystagmus measuring device that measures nystagmus data on the subject, and a medical interview result recognition unit that recognizes a result of a medical interview about dizziness with the subject;
any one of the aforementioned head-position sway measuring devices; and
a data storage unit that stores at least one of the hearing acuity threshold, the nystagmus data, and the medical interview result measured before a lapse of a predetermined length of time from when the head-position sway measuring device measured a head-position sway value, and the head-position sway value in association with the at least one of the hearing acuity threshold, the nystagmus data and the medical interview result.

The nystagmus data is used to evaluate the condition of dizziness, in addition to the head-position sway value. Since dizziness may be caused by an abnormality of inner ear organs (inner ear disorder), the inner ear disorder can also be grasped by examining the relationship between the data representing the condition of dizziness and the hearing acuity threshold. Also, since the actual impact of dizziness depends on how the subject feels, its combined use with medical interview with the subject may allow the impact of dizziness in daily life to be accurately grasped.

Therefore, in the case where the hearing acuity threshold, the nystagmus data, or the result of the medical interview about dizziness are stored as a series of data with the head-position sway value obtained by the head-position sway measuring device, reference to the stored data makes it possible to more accurately grasp the information representing the condition of dizziness and accurately grasp the condition of the inner ear disorder.

Also, in the biological information acquisition system of the present invention, the head-position sway measuring device is preferably a portable device.

This configuration allows the head-position sway measuring device to be installed, for example, even at subject's home. Thus, tests that would conventionally be performed only when the subject visited a hospital can be performed on a daily basis at subject's home or other places. This makes it possible to obtain detailed changes in the condition of the subject on a daily basis (i.e., on a continuous basis). As a result, the effects of treatment in clinical trials can be grasped with high accuracy, for example.

Also, the present invention is
a head-position sway measuring method of measuring a head-position sway value that is a value indicating a variation amount of sway of a head position of a subject, the method comprising the steps of:
instructing, by an opening/closing instruction unit that instructs the subject to open or close his or her eyes, the subject to do one of opening and closing eyes and then instructing the subject to do the other of opening and closing eyes successively or after a lapse of a predetermined length of time;
measuring, by a displacement measurement unit that measures a displacement of the head position of the subject, a first measurement value that is a measurement value of the displacement of the head position in a state where the opening/closing instruction unit instructs to open eyes and a second measurement value that is a measurement value of the displacement of the head position in a state where the opening/closing instruction unit instructs to close eyes; and
recognizing, by a sway recognition unit that recognizes the head-position sway value based on the measured displacement of the head position, the head-position sway value based on the first measurement value and the second measurement value related to the first measurement value.

### Brief Description of Drawings

FIG. 1 is an illustrative diagram showing a schematic configuration of a measurement system according to an embodiment.
FIG. 2 is a block diagram of a configuration of processing units included in the measurement system shown in FIG. 1.
FIG. 3 is a schematic view showing an example of time-series data of head position displacement acquired by the measurement system shown in FIG. 1.
FIG. 4 shows an example of an image used when the measurement system shown in FIG. 1 instructs a subject to open his or her eyes.
FIG. 5 shows an example of an image used when the measurement system shown in FIG. 1 instructs the subject to close his or her eyes.
FIG. 6 is a flowchart of processing to be executed during measurement of a head-position sway value of the subject using the measurement system shown in FIG. 1.

### Description of Embodiments

A measurement system S (biological information acquisition system) according to an embodiment will now be described with reference to the attached drawings.

### [Schematic Configuration of System]

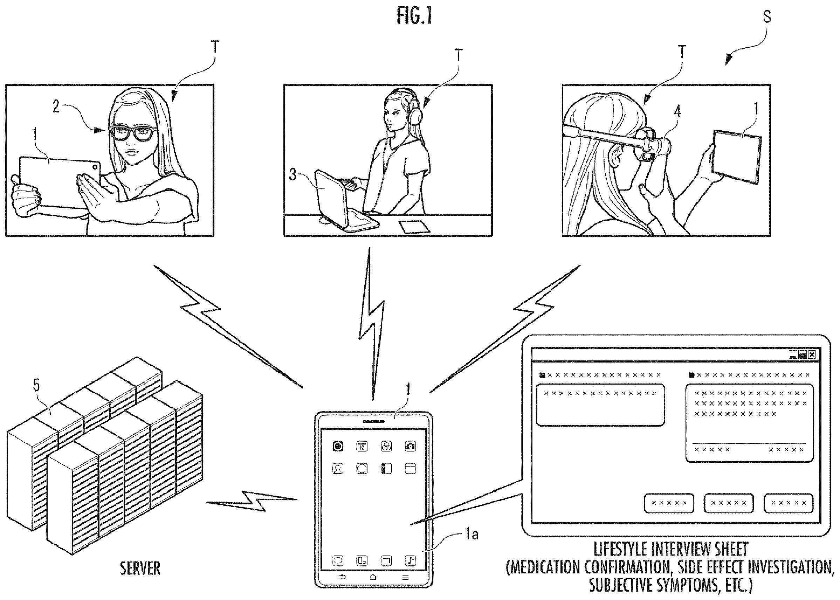

First, a schematic configuration of the measurement system S will be described referring to FIG. 1.

The measurement system S comprises a tablet 1, a head-position sway measuring instrument 2, an audiometer 3 (hearing acuity measuring device), Frenzel goggles 4 (nystagmus measuring device), and a server 5.

The head-position sway measuring instrument 2, the audiometer 3, and the Frenzel goggles 4 are each configured to be able to communicate with the tablet 1 by short-distance wireless communication or wired communication. In addition, the tablet 1 and the server 5 are configured to be able to communicate with each other via the Internet network, a public circuit, or the like.

Here, as described below, the head-position sway measuring instrument 2 and the Frenzel goggles 4 are configured to be wearable devices, and the audiometer 3 is configured to be a lightweight portable device. Thus, among the devices included in the measurement system S, those other than the server 5 are configured to be portable devices, which can be installed at the subject T's home or other places.

Therefore, with the measurement system S, tests that would conventionally be performed only when the subject T visited a hospital can be performed on a daily basis at the subject T's home or other places. This makes it possible to obtain detailed changes in the condition of the subject T on a daily basis (i.e., on a continuous basis). As a result, the effects of treatment in clinical trials can be grasped with high accuracy, for example.

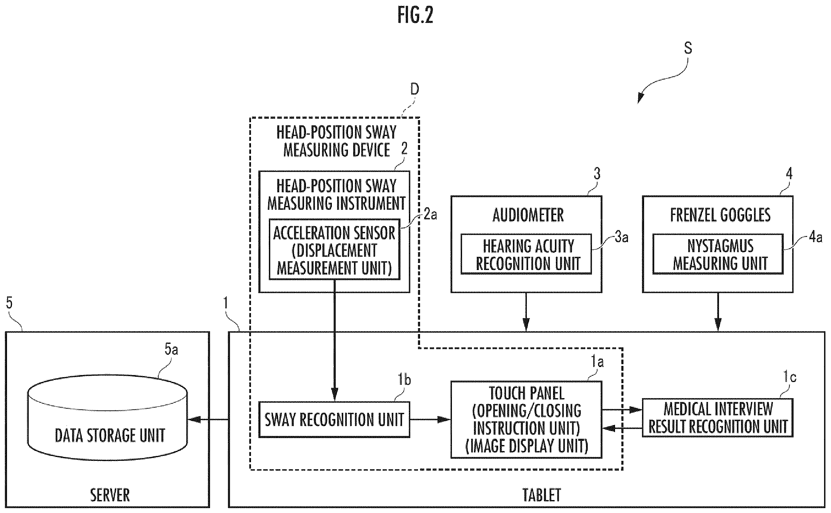

The tablet 1 comprises input and output units such as a touch panel 1a (opening/closing instruction unit and image display unit), a speaker (not shown in the drawing), and a microphone (not shown in the drawing). The tablet 1 cooperates with the head-position sway measuring instrument 2 to constitute a head-position sway measuring device D (see FIG. 2).

The head-position sway measuring instrument 2 is a wearable device in the form of goggles. The head-position sway measuring instrument 2 has an acceleration sensor 2a (displacement measurement unit) built in the frame to measure the displacement of the head position of the subject T (see FIG. 2). The displacement of the head position of the subject T measured by the acceleration sensor 2a is sent to the tablet 1.

The measurement system S is illustrated as if it uses the acceleration sensor 2a mounted on the head-position sway measuring instrument 2, which is a goggles-like wearable device, as a displacement measurement unit. However, the displacement measurement unit of the present invention should not necessarily be configured in such a way, but can be anything that can measure the displacement of the subject's head position.

Accordingly, for example, in the case where a displacement is measured using captured images, a camera installed in the room where the measurement is to be made, a camera mounted on a tablet or other device used for the measurement, or any other photographic device may be used as the displacement measurement unit. In addition, other sensors such as GPS may be mounted on the wearable device instead of the acceleration sensor, and that sensor may be used as the displacement measurement unit.

As described above, in the measurement system S, the tablet 1 and the head-position sway measuring instrument 2 cooperate with each other to constitute the head-position sway measuring device D. However, the head-position sway measuring device of the present invention should not necessarily be configured in such a way. For example, if the camera of the tablet can be used to measure the subject's head-position sway, the head-position sway measuring device may only consist of the tablet, omitting the independent head-position sway measuring instrument.

The audiometer 3 is configured to be a portable device. The audiometer 3 has a headphone that the subject T wears, a main unit connected to the headphone, and an input unit for the subject T to input whether or not he or she hears sound.

The Frenzel goggles 4 are configured to be a goggle-like wearable device. The Frenzel goggles 4 recognize the measured eye video as nystagmus data.

Note that the nystagmus measuring device of the present invention is not limited to the Frenzel goggles, but can be anything that can measure the nystagmus data of a subject. For example, instead of the Frenzel goggles, the nystagmus data may be measured using a so-called electronystagmograph. Also, instead of the Frenzel goggles, the camera mounted on the tablet may be used as a nystagmus measuring device and the nystagmus data may be measured using that camera.

The server 5 receives, via the tablet 1, the measurement values of head-position sway, which are the values indicating the variation amount of sway of the head position of the subject T measured by the head-position sway measuring device D, the audiogram generated by the audiometer 3, the nystagmus data measured by the Frenzel goggles 4, and the subject T's answers to the medical interview displayed on the tablet 1 (the result of the medical interview), all being associated with each other. In the server 5, these data are stored separately by date and time.

For the measurement system S, one measuring device, such as the head-position sway measuring device D, the audiometer 3, and the Frenzel goggles 4, is shown for one server 5.

However, the present invention is not limited to such a configuration, and multiple measuring devices may be provided for a single server. With such a configuration, it is possible to collect a large amount of data on the head position sway values, the hearing acuity thresholds, the nystagmus data, and the medical interview results, and make them into big data.

In the measurement system S, the processing units are distributed and placed in the head-position sway measuring instrument 2, the audiometer 3, the Frenzel goggles 4, and the server 5. However, such a configuration is merely illustrative, and the equipment to be installed with each processing unit may be selected as appropriate. For example, in the case where hearing acuity measurement and nystagmus-related video recording are performed on a tablet or other portable terminals, the hearing acuity recognition unit and the nystagmus measuring unit can be installed in that portable terminal.

### [Configuration of Processing Units]

Next, the processing units in the measurement system S will be described referring to FIG.s 2 to 5.

As shown in FIG. 2, the measurement system S has a sway recognition unit 1b and a medical interview result recognition unit 1c as functions (processing units) accomplished by the hardware configuration or program installed in the tablet 1.

The sway recognition unit 1b displays an instruction image 6 representing instructions about opening and closing the eyes to the subject T via the touch panel 1a (see FIG.s 4 and 5).

The sway recognition unit 1b also recognizes the measurement values obtained based on the acceleration sensor 2a of the head-position sway measuring instrument 2 (i.e., the measurement values of displacement of the head position of the subject T) on a time-series basis. The sway recognition unit 1b then recognizes head-position sway measurement values based on the measurement values and sends them to the server 5.

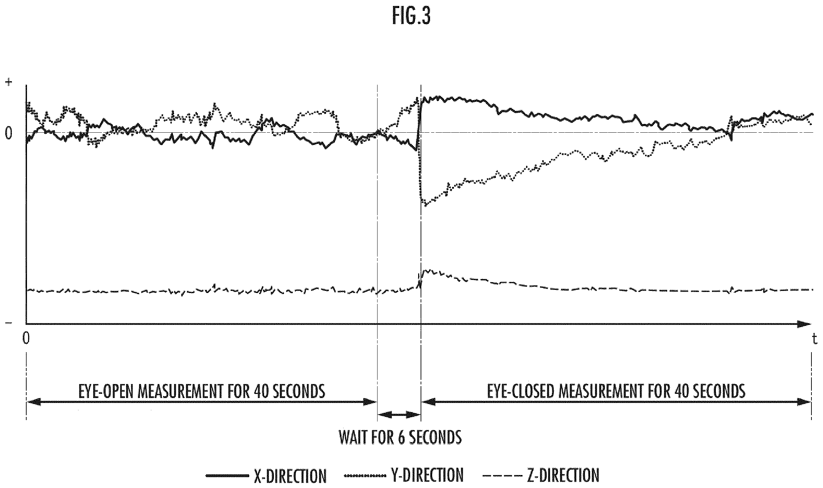

As described below, the measurement system S employs a first measurement value that is a measurement value of the displacement of the head position in the state where the tablet 1 instructs to open eyes and a second measurement value that is a measurement value of the displacement of the head position in the state where the tablet 1 instructs to close eyes, as the measurement values. Therefore, if those measurement values are plotted on the time-series basis, the data shown in FIG. 3, for example, can be obtained.

Here, the head-position sway value include, for example, the Romberg ratio calculated using the first and second measurement values. In the measurement system S, for each of the first and second measurement values, the Romberg ratio is calculated using the total length of the trajectory of the displacement of the head position during the measurement time or the area obtained from the trajectory of the displacement, and the Romberg ratio is used as a head position sway measurement value.

Note that a head-position sway value in the present invention is not limited to a Romberg ratio, but may be a value obtained based on a first measurement value and a second measurement value related to the first measurement value. For example, the value of the difference in time when a displacement exceeding a predetermined threshold occurred for each of the first and second measurement values may be used. If a displacement exceeding a predetermined threshold occurs for each of the first and second measurement values before recognition of the head-position sway value, these measurement values may be corrected using a predetermined calculation method.

The medical interview result recognition unit 1c stores data on matters that should be questioned as a lifestyle interview, such as medication confirmation, side effect investigation, subjective symptoms, and the like. Based on the data, the medical interview result recognition unit 1c presents the lifestyle interview sheet to the subject T via the touch panel 1a in a format that allows the subject to answer the interview (see FIG. 1). Upon reception of an answer from the subject T, the medical interview result recognition unit 1c recognizes the result of the medical interview and sends it to the server 5.

Note that answers to the medical interviews may be input not only through the touch panel 1a but also by voice input using the microphone mounted on the tablet 1.

The measurement system S comprises a hearing acuity recognition unit 3a as a function (processing unit) that is accomplished by a hardware configuration or program installed in the body unit of the audiometer 3.

The hearing acuity recognition unit 3a controls the frequency of the sound (pure sound) emitted through the headphones, generates an audiogram based on the measured hearing acuity threshold, and sends the audiogram to the server 5 via the tablet 1.

The measurement system S has a nystagmus measuring unit 4a as a function (processing unit) that is accomplished by a hardware configuration or program installed in the body unit of the Frenzel goggles 4.

The nystagmus measuring unit 4a recognizes the video data captured by the Frenzel goggles 4 as the nystagmus data, and sends the nystagmus data to the server 5 via the tablet 1.

The server 5 comprises a data storage unit 5a as a function (processing unit) that is accomplished by the hardware configuration or program installed.

The data storage unit 5a stores the head-position sway values, the audiograms, the nystagmus data, and the medical interview results that are recognized successively or within a predetermined length of time and sent from the tablet 1, all being associated with each other.

### [Details of instruction image]

By the way, as described above, in the measurement system S, via the touch panel 1a, an instruction image 6 representing instructions to the subject T about opening and closing of the eyes is displayed for the subject T. The instruction image 6 will be described in detail referring to FIG.s 4 and 5.

First, in general, during measurement of head-position sway, subjects tend to look at objects (such as a clock in a room) around them or not to look at anything, unless they are looking at some object. As a result, their gaze wanders, causing a risk of body deviation in the subject.

For this reason, the measurement system S is configured to display the instruction image 6 on the touch panel 1a of the tablet 1, and instructs the subject to open or close his or her eyes through the instruction image 6. Hence, the measurement system S makes the subject T naturally gaze at the instruction image 6, fixes the gaze of the subject T (and thus stabilizes his or her posture), and controls the body deviation of the subject T.

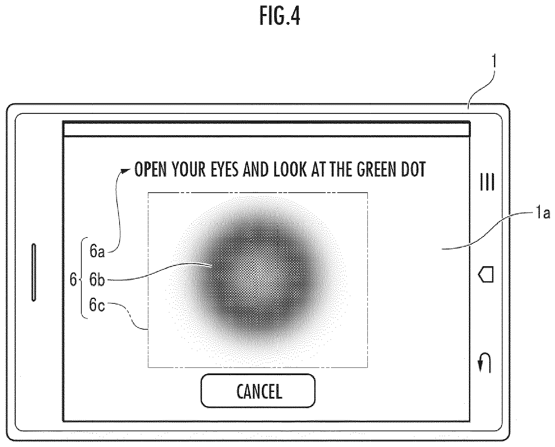

In the measurement system S, as shown in FIG. 4, the instruction image 6 displayed on the touch panel 1a consists of instruction text 6a, which is text for stating the details of the instruction, an indicator 6b (graphic image), which is the object of gaze of the subject T, and a background image 6c which is the background of the indicator 6b. As shown in FIG.s 4 and 5, according to the instruction given to the subject T at the time, the text of the instruction text 6a changes and switching between showing and hiding the indicator 6b and the background image 6c is performed.

In the layout of the example shown in the drawing, the indicator 6b and the background image 6c are placed below the instruction text 6a. However, the layout may be changed as necessary according to the recommended posture of the subject T during measurement.

If the boundary line of the indicator 6b is clear, under the influence of the boundary line, the sway control function of the subject T due to visual stimulus may be exerted excessively and the sway due to dizziness may be controlled.

Therefore, in the measurement system S, the boundary part of the indicator 6b adjacent to the background image 6c has a gradation of the saturation gradually changing from the periphery toward the center of the indicator 6b. This makes the boundary part ambiguous and prevents the subject T from exerting the sway control function excessively due to the stimulus from the boundary line.

The boundary part of the graphic image of the present invention does not necessarily have to have a gradation by gradually changing only the saturation, but may have a gradation by gradually changing the brightness, or brightness and saturation.

The graphic image of the present invention does not always include the boundary part with a gradation. For example, if the subject is an infant and priority should be given to fixating the gaze rather than preventing excessive exertion of the sway control function, an image of a character with a clear outline may be adopted as a graphic image.

The indicator 6b is a circular image of a graphic in which the saturation value of the indicator 6b increases from the periphery toward the center but, in the central area, decreases toward the center. Thus, the indicator 6b is an image of a graphic with not only a blurred periphery but also a blurred central area (i.e., an image that looks like a ring). The color of the background image 6c, which is the background of the indicator 6b, is white.

As a result, the gaze of the subject T is naturally guided to the center of the indicator 6b, but less likely to the background image 6c. Consequently, the gaze of the subject T can be firmly fixed and body deviation can be efficiently controlled.

Even if the shape of the graphic image of the present invention is not circular but polygonal, the same effect is achieved as long as at least one of the brightness and the saturation, in the peripheral area, increases toward the center, and in the central area, decreases toward the center. Here, "circular" shape includes not only circles but also ellipses and other generally circular shapes. Also, "polygonal" shape includes not only those with straight sides, but also those with the sides curved toward the periphery.

The background image of the present invention does not have to be white, and may be changed as necessary according to the color of the graphic image displayed in accordance with the background image, the brightness of the spot to be measured, and the like.

In addition, the graphic image of the present invention may be made into an image with a lightly colored central area as indicated by the indicator 6b, by making the brightness or saturation value of the central area different from that of the background image, or it may be even made into an annular image by changing the brightness or saturation value of the central area of the graphic image until it becomes the same as that of the background image.

By the way, if the indicator 6b is too small, the control of body deviation by gazing may not be sufficiently exerted. In contrast, if the indicator 6b is too large, the subject's posture may become unstable due to the intimidating feeling received from the image.

For this reason, in the measurement system S, the diameter of the indicator 6b is set to be greater than or equal to 2.5 cm and less than or equal to 10 cm. This value was determined referring to the statement in "An Investigation in the Role of the Visual Target in Stabilometry" in Equilibrium Research (Vol. 59(6), pp. 568-573, 2000).

This allows, for example, when the distance between the subject T and the touch panel 1a of the tablet 1 (i.e., the indicator 6b) is about one meter (i.e., when he or she has a general arm length and is holding by hand the device displaying the instruction image), appropriate body deviation control to be demonstrated, and the intimidating feeling given to the subject T can be softened to prevent postural instability.

Note that the size of the graphic image of the present invention is not limited to such a size. For example, the size of the graphic image may be changed as appropriate according to the posture recommended for the subject during measurement and the configuration of the device displaying the graphic image (for example, when the graphic image is displayed on the lens of a device on goggles).

### [Processing to be Executed]

Next, referring to FIG.s 1 and 2 and FIG.s 4 to 6, the processing to be executed in the measurement system S during measurement of head-position sway value of the subject T (i.e., the head-position sway measuring method) will be explained.

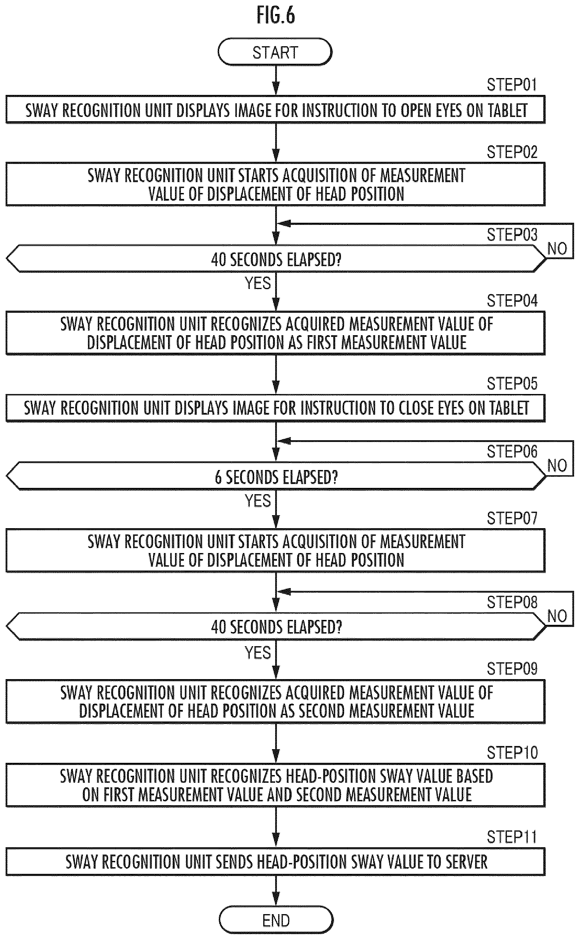

In this processing, first, the sway recognition unit 1b of the tablet 1 displays the instruction image 6 that gives the instruction to open the eyes, on the touch panel 1a of the tablet 1 (STEP01 in FIG. 6).

To be specific, as shown in FIG. 4, the sway recognition unit 1b displays on the touch panel 1a the instruction image 6 that includes the instruction text 6a stating "Open your eyes and look at the green dot", the indicator 6b, and the background image 6c.

The sway recognition unit 1b then starts acquiring the measurement values of the displacement of the head position of the subject T measured by the acceleration sensor 2a of the head-position sway measuring instrument 2 (STEP02 in FIG. 6).

The sway recognition unit 1b then determines whether or not 40 seconds have elapsed since the start of the acquisition of the measurement values of the displacement of the head position (STEP03 in FIG. 6).

If it is determined that 40 seconds have not elapsed (NO in STEP03), the sway recognition unit 1b continues to acquire the measurement values of the displacement of the head position and executes the processing of STEP03 again at a predetermined control cycle.

In contrast, if it is determined that 40 seconds have elapsed (YES in STEP03), the sway recognition unit 1b stops acquiring the measurement values of the displacement of the head position and recognizes the measurement values acquired after the instruction image 6 for the instruction to open the eyes was displayed, as the first measurement values (measurement values in the eye-open state) (STEP04 in FIG. 6).

Next, the sway recognition unit 1b displays an image for the instruction to close the eyes, on the touch panel 1a (STEP05 in FIG. 6).

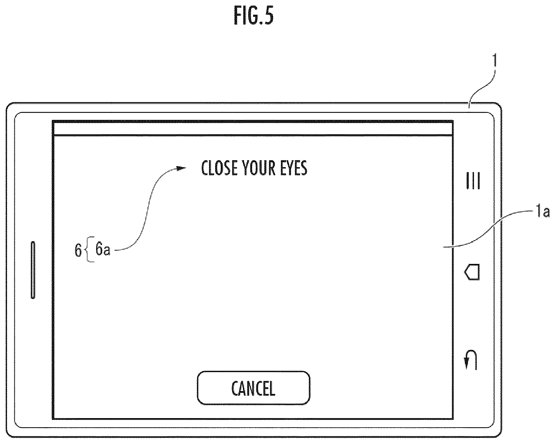

To be specific, as shown in FIG. 5, the sway recognition unit 1b hides the indicator 6b and the background image 6c, and displays only the instruction text 6a stating "Close your eyes" as the instruction image 6, on the touch panel 1a.

Next, the sway recognition unit 1b determines whether or not 6 seconds have elapsed since the start of display of the image for the instruction to close the eyes (STEP06 in FIG. 6).

This 6-second period is a transition period, which is the waiting time for the subject T to transition from the eye-open state to the eye-closed state. This transition period may be set as appropriate according to the physical characteristics of the subject T (e.g., age), measurement posture, and the like.

If it is determined that 6 seconds have not elapsed (NO in STEP06), the processing of STEP06 is executed again in a predetermined control cycle.

In contrast, if it is determined that 6 seconds have elapsed (YES in STEP06), the sway recognition unit 1b starts acquiring the measurement values of the displacement of the head position of subject T measured by the acceleration sensor 2a of the head-position sway measuring instrument 2 (STEP07 in FIG. 6).

Next, the sway recognition unit 1b determines whether or not 40 seconds have elapsed since the start of the acquisition of the measurement values of the displacement of the head position (STEP08 in FIG. 6).

If it is determined that 40 seconds have not elapsed (NO in STEP08), the sway recognition unit 1b continues to acquire the measurement values of the displacement of the head position and executes the processing of STEP08 again in a predetermined control cycle.

In contrast, if it is determined that 40 seconds have elapsed (YES in STEP06), the sway recognition unit 1b stops acquiring the measurement values of the displacement of the head position and recognizes the measurement values acquired after a lapse of 6 seconds after the instruction image 6 for the instruction to close the eyes was displayed, as the second measurement values (measurement values in the eye-closed state) (STEP09 in FIG. 6).

This processing produces data on the displacement of the head position of the subject T shown in FIG. 3.

The sway recognition unit 1b then recognizes the head-position sway value based on the recognized first and second measurement values (STEP 10 in FIG. 6).

To be specific, the sway recognition unit 1b calculates a value (e.g., Romberg ratio) set by the system designer based on the first and second measurement values, and recognizes that value as the head-position sway value.

The sway recognition unit 1b then sends the recognized head-position sway value to the server 5 (STEP11 in FIG. 6) and ends the current processing.

In the measurement system S, as well as the recognition of the head-position sway value following the aforementioned procedure, the measurement of the hearing acuity thresholds, the measurement of the nystagmus data, and the medical interview are performed before a predetermined length of time elapses from the time of the measurement of the head-position sway value (i.e., within a predetermined measurement period (e.g., 10 to 15 minutes)), and these data are associated with each other and stored in the data storage unit 5a of the server 5. The order of these measurements and the measurement period may be set as appropriate according to the type of device used for the measurement, the type of biological information to be measured, and the like.

As explained above, the head-position sway measuring device D in the measurement system S instructs the subject T to open or close his or her eyes via the touch panel 1a of the tablet 1, in conjunction with measuring the first measurement value and the second measurement value, which are the measurement values of the displacement of the head position in the state where the instruction to open or close the eyes is given, thereby recognizing the head-position sway value based on these measurement values.

At this time, the first measurement value and the second measurement value are measured after a predetermined length of time (i.e., a length of time predetermined by the designer) has elapsed. For this reason, the first measurement value and the second measurement value are necessarily measured with the same posture except for the eye-open or closed state.

The head-position sway value obtained by this head-position sway measuring device is based on the first measurement value and the second measurement value measured in this way, and are therefore more accurate than those obtained by conventional measurement devices. Therefore, reference to the head-position sway value obtained with this head-position sway measuring device D allows information indicating the condition of dizziness to be obtained more accurately.

The nystagmus data is used to evaluate the condition of dizziness, in addition to the head-position sway value. Since dizziness may be caused by an abnormality of inner ear organs (inner ear disorder), the inner ear disorder can also be grasped by examining the relationship between the data representing the condition of dizziness and the hearing acuity threshold. Also, since the actual impact of dizziness depends on how the subject T feels, its combined use with medical interview with the subject T may allow the impact of dizziness in daily life to be accurately grasped.

Therefore, as described above, the measurement system S is configured to store not only the head-position sway value but also the hearing acuity threshold, the nystagmus data, and the results of the medical interview about dizziness in the data storage unit 5a of the server 5 as a series of associated data. Hence, reference to the stored data makes it possible not only to accurately grasp the information representing the condition of dizziness, but also to accurately grasp the condition of the inner ear disorder.

### [Other Embodiments]

Although the embodiment shown in the drawings has been described above, the present invention is not limited to this embodiment.

For example, in the aforementioned embodiment, the measurement system S is configured to be able to store not only the head-position sway value of the subject T but also the hearing acuity threshold, the nystagmus data, and the result of the medical interview about dizziness as a series of associated data. This is because this measurement system S is configured to serve as the biological information acquisition system for the purpose of accurately grasping abnormalities of inner ear organs, such as hearing loss, as well as dizziness.

However, the biological information acquisition system of the present invention should not necessarily be configured in such a way, but in any way as long as it comprises not only a head-position sway measuring device but also at least one of a hearing acuity measuring device, a nystagmus measuring device, and a medical interview result recognition unit; and a data storage unit that stores these data in association with each other. The head-position sway measuring device may also be used as a stand-alone device.

In the aforementioned embodiment, the touch panel 1a of the tablet 1 is employed as an opening/closing instruction unit, and the instruction image 6 is displayed on the touch panel 1a in order to instruct the subject T to open or close his or her eyes.

However, the opening/closing instruction unit of the present invention should not necessarily be configured in this way but in any way as long as it can instruct the subject to open or close his or her eyes. Therefore, for example, an opening/closing instruction may be made using the voice from the speaker mounted in the tablet, or using both voice and images, thereby instructing the subject to open or close his or her eyes.

In the aforementioned embodiment, in the head-position sway measuring device D, the first measurement value is the measurement value of the displacement of the head position of the subject T in the state where the instruction to open the eyes is given via the touch panel 1a, and the second measurement value is the measurement value of the displacement of the head position of the subject T in the state where the instruction to close the eyes is given via the touch panel 1a. Based on this premise, the head-position sway measuring device D measures the first measurement value for 40 seconds and, after a transition period of 6 seconds, the second measurement value for 40 seconds.

However, the head-position sway measuring device of the present invention should not necessarily be configured in this way but in any way as long as it measures one of the first measurement value and the second measurement value, and then measures the other successively or after a lapse of a predetermined length of time.

Therefore, for example, the measurement period of the first measurement value, the measurement period of the second measurement value, and the transition period may be set as appropriate according to the state of the subject, the type of the measuring device, and the like. Also, after one of the first measurement value and the second measurement value is measured, the other may be measured without the transition period.

In the case where the first measurement value, which is measured in the eye-open state, is measured after the second measurement value, which is measured in the eye-closed state, is measured, the subject is preferably instructed to open his or her eyes by voice from the tablet's speaker or the like.

Further, in the aforementioned embodiment, the measurement values from the acceleration sensor 2a, which is a displacement measurement unit, are acquired at the sway recognition unit 1b as appropriate to recognize the first and second measurement values. However, this is not necessarily the case in the present invention. For example, the displacement measurement unit may perform measurement only in the period when the instruction to open or close the eyes is given.

By the way, in the aforementioned embodiment, the first measurement value is measured for 40 seconds with the subject T's eyes open, and after a lapse of 6 seconds of transition period, the second measurement value is measured for 40 seconds with the subject T's eyes closed.

Here, the instruction image 6 is displayed on the touch panel 1a of the tablet 1 while the first measurement value is being measured and during the transition period. This is to control the subject T's body deviation by instructing the subject T to open or close his or her eyes through the instruction image 6 so that the subject T can naturally gaze at the instruction image 6 and fix his or her gaze (and thus stabilize his or her posture), thereby controlling the body deviation of the subject T.

Besides, the measurement system S is configured to be able to perform accurate measurement by adding various innovations to the indicators 6b and the background image 6c included in the instruction image 6.

However, the effect of such an instruction image is achieved not only when one of the first measurement value and the second measurement value is measured and then the other is measured successively or after a lapse of a predetermined length of time. For example, such an effect is also achieved when the first measurement value is independently measured (i.e., when the measurement is simply made in the eye-open state).

### Reference Signs List

1...Tablet, 1a...Touch panel (opening/closing instruction unit, image display unit), 1b...Sway recognition unit, 1c...Medical interview result recognition unit, 2...Head-position sway measuring instrument, 2a...Acceleration sensor (displacement measurement unit), 3...Audiometer (hearing acuity measuring device), 3a...Hearing acuity recognition unit, 4...Frenzel goggles (nystagmus measuring device), 4a...Nystagmus measuring unit, 5...Server, 5a...Data storage unit, 6...Instruction image, 6a...Instruction text, 6b...Indicator (graphic image), 6c...Background image, D...Head-position sway measuring device, S...Measurement system (biological information acquisition system), T...Subject

## Claims

1. A head-position sway measuring device that measures a head-position sway value that is a value indicating a variation amount of sway of a head position of a subject, the device comprising:
an opening/closing instruction unit that instructs the subject to open or close his or her eyes;
a displacement measurement unit that measures a displacement of the head position of the subject; and
a sway recognition unit that recognizes the head-position sway value based on the measured displacement of the head position, wherein
the displacement measurement unit measures one of a first measurement value that is a measurement value of the displacement of the head position in a state where the opening/closing instruction unit instructs to open eyes and a second measurement value that is a measurement value of the displacement of the head position in a state where the opening/closing instruction unit instructs to close eyes, and then measures the other of the first measurement value and the second measurement value successively or after a lapse of a predetermined length of time, and
the sway recognition unit recognizes the head-position sway value based on the first measurement value and the second measurement value related to the first measurement value.

2. The head-position sway measuring device according to Claim 1, wherein the opening/closing instruction unit includes an image display unit that displays an instruction image representing an instruction to the subject to open or close his or her eyes.

3. The head-position sway measuring device according to Claim 2, wherein
the instruction image includes a predetermined graphic image and a background image that serves as a background of the graphic image, and
a boundary part of the graphic image adjacent to the background image has a gradation of at least one of brightness and saturation changing gradually from a periphery toward a center of the graphic image.

4. The head-position sway measuring device according to Claim 3, wherein
the graphic image is a circular or polygonal image, and
the value of the at least one of brightness and saturation of the graphic image increases from the periphery toward the center and, in a central area, decreases toward the center.

5. A biological information acquisition system comprising:
at least one of a hearing acuity measuring device that measures a hearing acuity threshold of the subject, a nystagmus measuring device that measures nystagmus data on the subject, and a medical interview result recognition unit that recognizes a result of a medical interview about dizziness with the subject;
the head-position sway measuring device according to any one of Claims 1 to 4; and
a data storage unit that stores at least one of the hearing acuity threshold, the nystagmus data, and the medical interview result measured before a lapse of a predetermined length of time from when the head-position sway measuring device measured a head-position sway value, and the head-position sway value in association with the at least one of the hearing acuity threshold, the nystagmus data and the medical interview result.

6. The biological information acquisition system according to Claim 5, wherein the head-position sway measuring device is a portable device.

7. A head-position sway measuring method of measuring a head-position sway value that is a value indicating a variation amount of sway of a head position of a subject, the method comprising steps of:
instructing, by an opening/closing instruction unit that instructs the subject to open or close his or her eyes, the subject to do one of opening and closing eyes and then instructing the subject to do the other of opening and closing eyes successively or after a lapse of a predetermined length of time;
measuring, by a displacement measurement unit that measures a displacement of the head position of the subject, a first measurement value that is a measurement value of the displacement of the head position in a state where the opening/closing instruction unit instructs to open eyes and a second measurement value that is a measurement value of the displacement of the head position in a state where the opening/closing instruction unit instructs to close eyes; and
recognizing, by a sway recognition unit that recognizes the head-position sway value based on the measured displacement of the head position, the head-position sway value based on the first measurement value and the second measurement value related to the first measurement value.
